# EUROPEAN PATENT SPECIFICATION

(11) **EP 3 218 066 B1**
(45) Date of publication and mention of the grant of the patent: **27.03.2019**
(21) Application number: 15794589.0
(22) Date of filing: 16.11.2015
(51) Int. Cl.: A61K 8/34, A61K 8/88, A61Q 13/00

(54) **LONG LASTING FRAGRANCE COMPOSITION**
LANGANHALTENDE DUFTSTOFFZUSAMMENSETZUNG
COMPOSITION DE PARFUM DE LONGUE DURÉE

(30) Priority: 14.11.2014 EP 14382451
(43) Date of publication of application: 20.09.2017
(73) Proprietor: Antonio Puig, S.A., 08902 Hospitalet de Llobregat (ES)
(72) Inventor: PANYELLA COSTA, David, E-08030 Barcelona (ES); VIDAL TASA, Jordi, E-08030 Barcelona (ES); GARCÍA ÀLVAREZ, Alejandro, E-08030 Barcelona (ES); ISNARDO IGLESIAS, David, E-08030 Barcelona (ES); MARTÍNEZ CORED, Mònica, E-08030 Barcelona (ES)
(74) Representative: ZBM Patents - Zea, Barlocci & Markvardsen
(86) International application number: PCT/EP2015/076666
(87) International publication number: WO 2016/075328

(56) References cited:
- WO-A1-95/24887
- WO-A1-03/096994
- US-A- 3 939 099
- DATABASE GNPD [Online] MINTEL; October 2007 (2007-10), "Deodorant", XP002740273, Database accession no. 790850
- DATABASE GNPD MINTEL; February 2014 (2014-02), "Gift Set", XP002740275, Database accession no. 2322113
- "Suncare compositions using amino hydroxy benzophenone derivatives", IP.COM JOURNAL, IP.COM INC., WEST HENRIETTA, NY, US, 3 October 2007 (2007-10-03), XP013122291, ISSN: 1533-0001
- DATABASE WPI Week 199347 Thomson Scientific, London, GB; AN 1993-374516 XP002740272, & JP H05 279236 A (SHISEIDO CO LTD) 26 October 1993 (1993-10-26)

## Description

The present composition relate to the field of cosmetics, particularly to a fragrance product which odour strength remains on the place of application for a long period of time.

### BACKGROUND ART

It is known that any fragrance composition, such as eau de toilettes (EDTs), eau de colognes (EDCs), eau de parfums (EDPs), aftershaves and others, tend to lose their odour strength after a relatively short period of time.

There is an interest in retaining the initial character and intensity of the fragrance on the skin for a long period of time, and various fragrance fixatives have been proposed, including film-forming polymers, in order to obtain a long lasting fragrance composition.

US6172037 discloses perfume-fixing substances comprising polyvinylpyrrolidone, hydroxypropyl cellulose and a hydrophobic oil.

EP1715841 discloses cosmetic compositions, particularly an EDT and an EDP, with a watertight fragrance comprising a fragrance substance, an acrylate octylacrylamide copolymer, and a hydrolysed jojoba ester.

WO2009053148 discloses a fragrancing composition comprising a fragrance substance, an amphiphilic copolymer of partially or completely neutralized 2-acrylamidomethylpropanesulphonic and an hydrophobic monomer in a cosmetically acceptable medium. This composition allows obtaining clear and fluid perfuming products while effectively increasing the staying power or the fragrance.

Despite the possible ways known in the prior art to fix a perfume component on the skin for a long time, the provision of fragrance compositions allowing to extent the time the perfume lasts on the skin goes on being an active field of research.

### SUMMARY OF THE INVENTION

Inventors have found that the incorporation of a polymer of the type polyamide-3 or polyamide-4 in a hydroalcoholic composition, allows obtaining a clear, stable, and long lasting fragrance composition. Thanks to the type of polymers used in the composition, the perfume component remains on the skin for a longer time than the known compositions, such as some EDTs and EDPs, disclosed in the prior art. The long lasting fragrance effect is found even when the polymer is used at low concentrations. The fact that these polymers can be incorporated in the compositions of the invention yielding to clear compositions is unexpected, since these polymers are poorly watersoluble (cf. IP.com Journal, 2012, Vol.12, Issue 3A, in which only compositions wherein the solvent is ethanol are disclosed). The skilled in the art would not contemplate their use in fragrance compositions, such as EDTs, EDPs, EDCs, aftershaves or others, since water is a component in these type of compositions.

Thus, a first aspect of the invention is a composition comprising a mixture of a) a polyalkyleneoxy terminated polyamide, b) a perfumed solution, and, c) optionally, ethylhexylglycerin, wherein the perfumed solution comprises a cosmetically acceptable medium and a fragrance substance, wherein the cosmetically acceptable medium comprises at least one volatile alcohol, water and, optionally, one or more cosmetically acceptable topical excipients, and wherein the amount of water in the composition is at least from 4 wt%, and wherein the amounts of fragrance substance, polyamide, water, and at least one volatile alcohol are such that the different components are co-dissolved in the solvent system formed by water and at least one volatile alcohol giving rise to a clear and stable solution, with the proviso that when the composition does not comprise ethylhexylglycerin then the amount of polyamide is from 0.1 wt% to 2 wt% and the amount of perfumed solution is from 99,9 wt% to 98,0 wt%; and when the composition comprises ethylhexylglycerin, then the amount of polyamide is from 0.1 wt% to 4 wt%, the amount of perfumed solution is from 99,8 wt% to 84,0 wt%, and the amount of ethylhexylglycerin is of 0.1 wt% to 12 wt%.

### DETAILED DESCRIPTION OF THE INVENTION

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

The term "cosmetically acceptable" or "dermatological acceptable" which is herein used interchangeably refers to that excipients or carriers suitable for use in contact with human skin without undue toxicity, incompatibility, instability, among others.

The term "fragrance substance", as used herein, means any natural or synthetic substance or substances used solely to impart an odour to a cosmetic product. Fragrance substances are characterized by having a vapour pressure below atmospheric pressure at ambient temperatures. A fragrance substance may include single compounds and mixtures of compounds. Specific examples of such compounds include materials such as alcohols, aldehydes, ketones, esters, acetates, terpenic hydrocarbons, as well as synthetic or natural oils, including essential oils.

The compositions of the present invention can comprise or consist of, the components described herein.

All percentages used herein are by weight of the total composition, unless otherwise designated. As used herein, the term "wt%" means weight percent.

As mentioned above, an aspect of the invention relates to a long lasting fragrance composition comprising a polyalkyleneoxy terminated polyamide, a perfumed solution comprising a cosmetically acceptable medium and a fragrance substance, and, optionally, ethylhexylglycerin, wherein some of the components are in the specific weight percentages indicated above.

General examples of polyalkyleneoxy terminated polyamides include resins identified by the INCI names polyamide-3 and polyamide-4, and marketed under Sylvaclear and/or OleoCraft tradenames, available for instance from Arizona Chemical or Croda. Polyamide-3 and polyamide-4 can be obtained by the condensation of dilinoleic acid, ethylenediamine, polypropylene glycol diamine end-capped with polyethylene glycol-polypropylene glycol (PEG-PPG)-32/10 aminopropyl methyl ether.

The cosmetically acceptable medium in accordance with the present invention comprises at least one volatile alcohol and water.

The amounts of fragrance substance, polyamide, water, and at least one volatile alcohol are such that the different components are co-dissolved in the solvent system formed by water and at least one volatile alcohol giving rise to a clear and stable solution. In particular, the amount of water that can be incorporated into the system depends on the nature of the fragrance composition and the percentage of fragrance substance.

The volatile alcohols in accordance with the present invention are preferably chosen from C1-C5 lower monoalcohols, and can be chosen from methanol, ethanol, propanol, isopropanol, n-butanol, isobutanol, and t-butanol. Particularly, the at least one volatile alcohol is ethanol.

Fragrance substances that may be used in the composition of the invention include fragrances and aromas of natural or synthetic origin, and mixtures thereof.

Fragrances and aromas of natural origin include, without being limited to, extracts of flowers (lily, lavender, rose, jasmine, ylang-ylang), of stems and of leaves (patchouli, geranium, bitter leaf), of fruits (coriander, anise, cumin, juniper), of fruit peel (bergamot, lemon, orange), of roots (angelica, celery, cardamom, iris, sweet flag), of wood (pinewood, sandalwood, lignum vitae, pink cedar), of herbs and grasses (tarragon, lemongrass, sage, thyme), of needles and branches (spruce, fir, pine, dwarf pine), of resins and of balsams (galbanum, gum elemi, gum benzoin, myrrh, frankincense, opopanax).

Fragrance substance of synthetic origin include, without being limited to, compounds of ester, ether, aldehyde, ketone, aromatic alcohol and hydrocarbon type. Examples of esters include, without being limited to, benzyl acetate, benzyl benzoate, phenoxyethyl isobutyrate, p-tert-butylcyclohexyl acetate, citronellyl acetate, citronellyl formate, geranyl acetate, linalyl acetate, dimethylbenzylcarbinyl acetate, phenylethyl acetate, linalyl benzoate, benzyl formate, ethylmethylphenyl glycinate, alkylcyclohexyl propionate, styrallyl propionate, and benzyl salicylate. Examples of ethers include, without being limited to, benzyl ethyl ether. Examples of aldehydes include, without being limited to, linear alkanals containing from 8 to 18 carbon atoms, citral, citronellal, cintronellyloxyacetaldehyde, cyclamenaldehyde, hydroxycitronellal, lilial and bourgeonal. Examples of ketones include, without being limited to, ionones such as alpha-isomethylionone and methyl cedryl ketone. Examples of aromatic, and in particular terpenic, alcohols, include, without being limited to, anethol, citronellol, eugenol, isoeugenol, geraniol, linalol, phenylethyl alcohol and terpineol. Examples of hydrocarbons include, without being limited to, terpenes. These compounds are often in the form of a mixture of two or more of these odourous substances.

Moreover, it is also possible to use essential oils, and components of aromas, such as, for example, sage, camomile, clove, Melissa, mint, cinnamon leaf, lime blossom, juniper, vetiver, frankincense, galbanum, labolanum and lavandin essences.

The fragrances mentioned above are all commercially available.

In a particular embodiment, optionally in combination with one or more features of the particular embodiments defined above, the composition of the invention does not comprise ethylhexylglycerin, i.e. ethylhexylglycerin is absent in the composition, and the amount or water in the fragrance composition is at least from 4 wt% to 18 wt%.

In a more particular embodiment, the composition of the invention does not comprise ethylhexylglycerin, and the polyamide is selected from a polyalkyleneoxy terminated polyamide. More particularly, the polyamide is selected from a polyamide-3 or a polyamide-4, such as OleoCraft MP-30, OleoCraft MP-32, OleoCraft HP-31, OleoCraft HP-33, SYLVASOL 80, Sylvaclear AF1900V, Sylvaclear PE1800V, Sylvaclear PA1200V, and Sylvaclear WF1500V.

In another particular embodiment of the composition not comprising ethylhexylglycerin, optionally in combination with one or more features of the particular embodiments defined above, in the perfumed solution the amount of cosmetically acceptable medium is from 99,9 wt% to 60,0 wt%, the at least one fragrance substance is from 0.1 wt% to 40.0 wt%, and in the cosmetically acceptable medium the amount of ethanol is from 60 wt% to 99.9 wt%, the amount of water is from 30 wt% to 0,1 wt%, and the amount of the at least one or more cosmetically acceptable topical excipients is from 0 wt% to 10 wt%.

In another particular embodiment of the composition not comprising ethylhexylglycerin, the polyamide is OleoCraft HP-33 in an amount of 0.1 wt% and the amount of water is of up to 14 wt%. In another particular embodiment of the composition not comprising ethylhexylglycerin, the polyamide is OleoCraft HP-33 in an amount of 1.4 wt% and the amount of water is of up to 8 wt%. In another particular embodiment of the composition not comprising ethylhexylglycerin, the polyamide is OleoCraft HP-31 in an amount of 0.1 wt% and the amount of water is of up to 18 wt%. In another particular embodiment of the composition not comprising ethylhexylglycerin, the polyamide is OleoCraft HP-31 in an amount of 1.,4 wt% and the amount of water is of up to 8 wt%. In another particular embodiment of the composition not comprising ethylhexylglycerin, the polyamide is OleoCraft MP-32 in an amount of 0.1 wt% and the amount of water is of up to 12 wt%. In another particular embodiment of the composition not comprising ethylhexylglycerin, the polyamide is OleoCraft MP-32 in an amount of 1.4 wt% and the amount of water is of up to 6 wt%.

In a particular embodiment, the composition of the invention comprises ethylhexylglycerin and the polyamide is a polyalkyleneoxy terminated polyamide. More particularly, the polyamide is selected from a polyamide-3 or a polyamide-4, such as OleoCraft MP-30, OleoCraft MP-32, OleoCraft HP-31, OleoCraft HP-33, SYLVASOL 80, Sylvaclear AF1900V, Sylvaclear PE1800V, Sylvaclear PA1200V, and Sylvaclear WF1500V.

It has been surprisingly found that when ethylhexylglycerin is used, the solubility of the non-water soluble components in the cosmetically acceptable medium is even higher, and so, a higher amount of polyamide, water or both can be included in the composition.

In another particular embodiment of the composition comprising ethylhexylglycerin, the amount of ethylhexylglycerin is from 0.1 to 12% by weight.

In another particular embodiment of the composition comprising ethylhexylglycerin, optionally in combination with one or more features of the particular embodiments of the composition comprising ethylhexylglycerin defined above, in the perfumed solution the amount of cosmetically acceptable medium is from 99.8 wt% to 84 wt%, the at least one fragrance substance is from 0.1 wt% to 40.0 wt%, and in the cosmetically acceptable medium the amount of ethanol is from 60 wt% to 99.9 wt%, the amount of water is from 30 wt% to 0,1 wt%, and the amount of the at least one or more cosmetically acceptable topical excipients is from 0 wt% to 10 wt%.

In another particular embodiment, the composition comprises 11 wt% of ethylhexylglycerin, 3.6 wt% OleoCraft HP-33, and up to 13 wt% of water. In another particular embodiment, the composition comprises 11 wt% of ethylhexylglycerin, 3.6 wt% of OleoCraft HP-31, and up to 11 wt% of water. In another particular embodiment, the composition comprises 11 wt% of ethylhexylglycerin, 3.6 wt% of OleoCraft MP-32, and up to 11 wt% of water.

In another particular embodiment, the composition of the invention can further comprise one or more appropriate topical cosmetically acceptable excipient or carrier selected from colour stabilizers, colorants, additives, UV filters, viscosity enhancers, peptizers and antioxidants. In particular, the amount of the one or more excipients or carriers is up to a 10% by weight.

The composition of the invention can be used in cosmetic products such as perfumes (eau de parfum, EDP), colognes (eau de cologne, EDC) and eau de toilettes (EDT). Additionally, it can also be used in other cosmetic products such as aftershaves and others. Accordingly, in a particular embodiment, the composition of the invention comprises an amount of water from at least 4 to 18% by weight.

Examples of appropriate colorants include, without being limited to, caramel, Yellow 5, Acid Blue 9/Blue 1, Green 5, Green 3/Fast Green FCF 3, Orange 4, Red 4/Food Red 1, Yellow 6, Acid Red 33/Food Red 12, Red 40, cochineal carmine (CI 15850, CI 75470), Ext. Violet 2, Red 6-7, Ferric Ferrocyanide, Ultramarines, Acid Yellow 3/Yellow 10, Acid Blue 3, Yellow 10. These colorants are commercially available.

Examples of appropriate additives normally used in the fragrance field include, without being limited to, cosmetic and dermatological active agents, emollients such as sweet almond oil and apricot kernel oil, moisturizers such as glycerol, calmatives such as [alpha]-bisabolol, allantoin, aloe vera; vitamins, essential fatty acids, insect repellants, propellants, fillers, pearlescent agents, flakes, dyestuffs that are soluble in the support of the composition, and mixtures thereof. These moisturizing substances are commercially available.

Examples of UV filters include, without being limited to, octocrylene, ethylhexyl triazone, diethylhexyl butamido triazone, ethylhexyl methoxycinnamate, butyl methoxydibenzoylmethane, ethylhexyl salicylate, diethylamino hydroxybenxoyl hexyl benzoate, sodium benzotriazolyl butylphenol sulfonate, and benzotriazolyl dodecyl p-cresol. These UV filters are commercially available.

Examples of appropriate viscosity enhancers include, without being limited to, carbomer, acrylates/c10-30 alkyl acrylate crosspolymer, polyacrylate crosspolymer-6, Hydroxyethyl Acrylate/Sodium Acryloyldimethyl Taurate Copolymer. These viscosity enhancers are commercially available.

Examples of appropriate antioxidants include, without being limited to, butylated hydroxytoluene (BHT), tetrabutyl ethylidinebisphenol, tetrasodium glutamate diacetate, diethylhexyl syringylidene malonate, pentaerythrityl tetradi-butyl hydroxyhydrocinnamate, tetrasodium glutamate diacetate, tocopherols such as vitamin E and its derivatives such as tocopheryl acetate. These antioxidants are commercially available.

Examples of appropriate light stabilizers or quenchers include, without being limited to, tris-(tetramethylhydroxypiperidinol) citrate and their variants. These stabilizers or quenchers are commercially available.

The composition of the invention can include one or more particles. The term "particle" refers to a discrete portion of material that has mass and dimension being elements of a suspension. In an embodiment of the invention, the particles can modify the visual appearance of the composition. Examples of these particles includes, without being limited to, sparkling particles such as glitter; reflective particles; encapsulated particles; microsphere enclosing colour particles,

The composition of the invention can be used in different devices that are well known to a person skilled in the art and comprise pump bottles or sprays, aerosol containers comprising a propellant and also aerosol pumps that use compressed air as a propellant.

The composition of the invention can be prepared by conventional methods known to those skilled in the art, such as the one described in the example. Throughout the description and claims the word "comprise" and variations of the word, are not intended to exclude other technical features, additives, components, or steps. Furthermore, the word "comprise" encompasses the case of "consisting of". The following examples are provided by way of illustration, and they are not intended to be limiting of the present invention. Furthermore, the present invention covers all possible combinations of particular and preferred embodiments described herein.

### EXAMPLES

### EXAMPLE 1

An eau de toilette (composition 1) was prepared by mixing a 12 wt% of fragrance, a 80 wt% of ethanol, and a 8 wt% of deionised water. This composition was used as a control.

Similarly, compositions 2 to 4 shown in the Table 1 below were prepared by incorporating a 1wt% of Oleocraft MP32, a 1wt% of Oleocraft HP31, and a 1 wt% of Dermacryl-79, respectively.

**Table 1**

| | |
|---|---|
| Composition 1 | Ethanol 80% |
| | Fragrance 12% |
| | Deionised water 8% |
| Composition 2 | Composition 1 + 1% Oleocraft MP32 |
| Composition 3 | Composition 1 + 1% Oleocraft HP31 |
| Composition 4 | Composition 1 + 1% Dermacryl-79 |

Composition 1 is a control composition of an eau de toilette without comprising any film forming polymer. Compositions 2 and 3 are examples of the invention. Composition 4 is a comparative example with a film forming not included among the ones used in the compositions of the invention.

### EXAMPLE 1 - Olfactory test

A circumference is delimited in the left mid-forearm of each subject. Then 1 µl of each one of the assayed composition is applied in the delimited areas of different subjects.

An assessment panel of 25 trained members accustomed to evaluate perfumes based on olfactory evidences was chosen. At 6 hours of the application of the perfumed composition, every panellist smelled the delimited areas in the mid-forearm of the subjects. Then, they indicated whether they smelled the applied perfume or not. In the cases they still smelled the perfume, they classified the intensity of mild or very mild. The results of each of the 25 panellists were assigned a value of 2 if they mildly smelled the perfume, of 1 if very mildly smelled the perfume, and of 0 if they did not smelled the perfume at all. The number of points for the control composition (composition 1) and for the assayed compositions (compositions 2-4) were summed up and compared for significance.

Results are shown in Table 2 below.

**Table 2**

| Perception | Value | Composition | | | |
|---|---|---|---|---|---|
| | | 1 | 2 | 3 | 4 |
| No smell | 0 | 8 | 2 | 6 | 2 |
| very mild smell | 1 | 6 | 8 | 1 | 12 |
| mild smell | 2 | 11 | 15 | 18 | 11 |
| TOTAL | | 28 | 38 | 37 | 34 |

The results show that the compositions of the invention allow the sustained delivery of the fragrance for a longer time than the one of the comparative example. Additionally, it is shown that the effect is also improved with respect to a comparative composition comprising Dermacryl-79, a film forming polymer known to be used in this kind of compositions.

The test was repeated several times in different days and on different subjects, being the results obtained very similar. So, this test is suitable to assess whether a composition, with or without specific additives, remains on the skin during a longer time or not.

### REFERENCES CITED IN THE APPLICATION

1. US6172037
2. EP1715841
3. WO2009053148
4. IP.com Journal, 2012, Vol. 12, Issue 3A, 9 pages

## Claims

1. A composition comprising a mixture of a) a polyalkyleneoxy terminated polyamide, b) a perfumed solution, and, c) optionally, ethylhexylglycerin, wherein the perfumed solution comprises a cosmetically acceptable medium and a fragrance substance,
wherein the cosmetically acceptable medium comprises at least one volatile alcohol, water and, optionally, one or more cosmetically acceptable topical excipients,
wherein the amount of water in the composition is at least from 4 wt%, and
wherein the amounts of fragrance substance, polyamide, water, and at least one volatile alcohol are such that the different components are co-dissolved in the solvent system formed by water and at least one volatile alcohol giving rise to a clear and stable solution,
with the proviso that when the composition does not comprise ethylhexylglycerin then the amount of polyamide is from 0.1 wt% to 2 wt% and the amount of perfumed solution is from 99,9 wt% to 98,0 wt%; and when the composition comprises ethylhexylglycerin, then the amount of polyamide is from 0.1 wt% to 4 wt%, the amount of perfumed solution is from 99,8 wt% to 84,0 wt%, and the amount of ethylhexylglycerin is of 0.1 wt% to 12 wt%.

2. The composition according to claim 1, wherein the at least one volatile alcohol is ethanol.

3. The composition according to any one of claims 1 or 2, wherein the ethylhexylglycerin is absent and the polyamide is a polyamide-3 or a polyamide-4.

4. The composition according to claim 3, wherein in the perfumed solution the amount of cosmetically acceptable medium is from 99,9 wt% to 60,0 wt%, the at least one fragrance substance is from 0.1 wt% to 40.0 wt%, and in the cosmetically acceptable medium the amount of ethanol is from 60 wt% to 99.9 wt%, the amount of water is from 30 wt% to 0,1 wt%, and the amount of the one or more appropriate topical cosmetically acceptable excipients or carriers is from 0 wt% to 10 wt%.

5. The composition according to claims 1 or 2 comprising ethylhexylglycerin, wherein the polyamide is a polyamide-3 or a polyamide-4.

6. The composition according to claim 5, wherein the amount of ethylhexylglycerin is from 0.1 wt% to 12 wt%.

7. The composition according to claim 6, wherein in the perfumed solution the amount of cosmetically acceptable medium is from 99,8 wt% to 84,0 wt%, the at least one fragrance substance is from 0.1 wt% to 40.0 wt%, and in the cosmetically acceptable medium the amount of ethanol is from 60 wt% to 99.9 wt%, the amount of water is from 30 wt% to 0,1 wt%, and the amount of the one or more appropriate topical cosmetically acceptable excipients or carriers is from 0 wt% to 10 wt%.

8. The composition according to any one of claims 1 to 7, further comprising one or more appropriate topical cosmetically acceptable excipients or carriers selected from colour stabilizers, colorants, additives, UV filters, viscosity enhancers, peptizers and antioxidants.

9. The composition according to any one of claims 1 to 8, wherein the amount of water is from at least 4 wt% to 18% wt%.

10. The composition according to any one of claims 1 to 9, in the form of an eau de toilette, eau de cologne, eau de parfum, or aftershave.

## Patentansprüche

1. Eine Zusammensetzung umfassend eine Mischung aus a) einem polyalkylenoxyterminierten Polyamid, b) einer parfümierten Lösung, und c), wahlweise, Ethylhexylglycerin,
wobei die parfümierte Lösung ein kosmetisch akzeptables Mittel und einen Duftstoff umfasst,
wobei das kosmetisch akzeptable Mittel mindestens einen flüchtigen Alkohol, Wasser und, wahlweise, einen oder mehrere kosmetisch akzeptable topische Hilfsstoffe umfasst,
wobei die Menge an Wasser in der Zusammensetzung von mindestens 4 Gew.-% ist, und
wobei die Mengen an Duftstoff, Polyamid, Wasser und mindestens einem flüchtigen Alkohol so sind, dass die verschiedenen Komponenten im aus Wasser und mindestens einem flüchtigen Alkohol bestehenden Lösungsmittelsystem zusammen gelöst werden, wodurch eine klare und stabile Lösung entsteht,
mit der Maßgabe, dass, wenn die Zusammensetzung kein Ethylhexylglycerin enthält, die Menge an Polyamid von 0,1 Gew.-% bis 2 Gew.-% reicht und die Menge an parfümierter Lösung von 99,9 Gew.-% bis 98,0 Gew.-% reicht; und wenn die Zusammensetzung Ethylhexylglycerin enthält, die Menge an Polyamid von 0,1 Gew.-% bis 4 Gew.-% reicht, die Menge an parfümierter Lösung von 99,8 Gew.-% bis 84,0 Gew.-% reicht, und die Menge an Ethylhexylglycerin von 0,1 Gew.-% bis 12 Gew.-% reicht.

2. Die Zusammensetzung nach Anspruch 1, wobei der mindestens eine flüchtige Alkohol Ethanol ist.

3. Die Zusammensetzung nach einem der Ansprüche 1 oder 2, wobei das Ethylhexylglycerin nicht vorhanden ist und das Polyamid ein Polyamid-3 oder ein Polyamid-4 ist.

4. Die Zusammensetzung nach Anspruch 3, wobei in der parfümierten Lösung die Menge an kosmetisch akzeptablem Mittel von 99,9 Gew.-% bis 60,0 Gew.-% reicht, der mindestens eine Duftstoff von 0,1 Gew.-% bis 40,0 Gew.-% vorhanden ist und im kosmetisch akzeptablen Mittel die Menge an Ethanol von 60 Gew.-% bis 99,99 Gew.-% reicht, die Menge an Wasser von 30 Gew.-% bis 0,1 Gew.-% reicht und die Menge an dem einen oder den mehreren geeigneten topischen kosmetisch akzeptablen Hilfsstoffen oder Trägersubstanzen von 0 Gew.-% bis 10 Gew.-% reicht.

5. Die Zusammensetzung nach einem der Ansprüche 1 oder 2 umfassend Ethylhexylglycerin, wobei das Polyamid ein Polyamid-3 oder ein Polyamid-4 ist.

6. Die Zusammensetzung nach Anspruch 5, wobei die Menge an Ethylhexylglycerin von 0,1 Gew.-% bis 12 Gew.-% reicht.

7. Die Zusammensetzung nach Anspruch 6, wobei in der parfümierten Lösung die Menge an kosmetisch akzeptablem Mittel von 99,8 Gew.-% bis 84,0 Gew.-% reicht, der mindestens eine Duftstoff von 0,1 Gew.-% bis 40,0 Gew.-% vorhanden ist und im kosmetisch akzeptablen Mittel die Menge an Ethanol von 60 Gew.-% bis 99,99 Gew.-% reicht, die Menge an Wasser von 30 Gew.-% bis 0,1 Gew.-% reicht und die Menge an dem einen oder den mehreren geeigneten topischen kosmetisch akzeptablen Hilfsstoffen oder Trägersubstanzen von 0 Gew.-% bis 10 Gew.-% reicht.

8. Die Zusammensetzung nach einem der Ansprüche 1 bis 7, weiterhin umfassend einen oder mehrere geeignete topische kosmetisch akzeptable Hilfsstoffe oder Trägersubstanzen ausgewählt aus Farbstabilisierungsmitteln, Farbstoffen, Zusatzstoffen, UV-Filtern, Mitteln zur Verbesserung der Viskosität, Peptisatoren und Antioxidationsmitteln.

9. Die Zusammensetzung nach einem der Ansprüche 1 bis 8, wobei die Menge an Wasser von mindestens 4 Gew.-% bis 18 Gew.-% reicht.

10. Die Zusammensetzung nach einem der Ansprüche 1 bis 9, in Form von *Eau de Toilette*, *Eau de Cologne*, *Eau de Parfum*, oder *Aftershave.*

## Revendications

1. Une composition comprenant un mélange de a) une polyamide à terminaison polyalkylénoxy, b) une solution parfumée, et, c), facultativement, éthylhexylglycérine, dans laquelle la solution parfumée comprend un milieu cosmétiquement acceptable et une fragrance,
dans laquelle le milieu cosmétiquement acceptable comprend au moins un alcool volatil, de l'eau et, facultativement, un ou plusieurs excipients topiques cosmétiquement acceptables,
dans laquelle la quantité d'eau dans la composition est au moins de 4 % en poids, et dans laquelle les quantités de fragrance, polyamide, eau, et au moins un alcool volatil sont telles que les différents composants sont co-dissous dans le système de solvants formé par l'eau et au moins un alcool volatil, ce qui donne une solution claire et stable, à condition que lorsque la composition ne comprend pas d'éthylhexylglycérine, alors la quantité de polyamide est de 0,1 % en poids à 2 % en poids et la quantité de solution parfumée est de 99,9 % en poids à 98,0 % en poids ; et lorsque la composition comprend d'éthylhexylglycérine, alors la quantité de polyamide est de 0,1 % en poids à 4 % en poids, la quantité de solution parfumée est de 99,8 % en poids à 84,0 % en poids, et la quantité d'éthylhexylglycérine est de 0,1 % en poids à 12 % en poids.

2. La composition selon la revendication 1, dans laquelle l'au moins un alcool volatil est l'éthanol.

3. La composition selon l'une quelconque des revendications 1 ou 2, dans laquelle l'éthylhexylglycérine n'est pas présente et la polyamide est une polyamide-3 ou une polyamide-4.

4. La composition selon la revendication 3, dans laquelle dans la solution parfumée la quantité de milieu cosmétiquement acceptable est de 99,9 % en poids à 60,0 % en poids, l'au moins une fragrance est de 0,1 % en poids à 40,0 % en poids, et dans le milieu cosmétiquement acceptable la quantité d'éthanol est de 60 % en poids à 99,9 % en poids, la quantité d'eau est de 30 % en poids à 0,1 % en poids, et la quantité de l'un ou les plusieurs excipients ou véhicules cosmétiquement acceptables topiques appropriés est de 0 % en poids à 10 % en poids.

5. La composition selon les revendications 1 ou 2 comprenant de l'éthylhexylglycérine, dans laquelle la polyamide est une polyamide-3 ou une polyamide-4.

6. La composition selon la revendication 5, dans laquelle la quantité d'éthylhexylglycérine est de 0,1 % en poids à 12 % en poids.

7. La composition selon la revendication 6, dans laquelle dans la solution parfumée la quantité de milieu cosmétiquement acceptable est de 99,8 % en poids à 84,0 % en poids, l'au moins une fragrance est de 0,1 % en poids à 40,0 % en poids, et dans le milieu cosmétiquement acceptable la quantité d'éthanol est de 60 % en poids à 99,9 % en poids, la quantité d'eau est de 30 % en poids à 0,1 % en poids, et la quantité de l'un ou les plusieurs excipients ou véhicules cosmétiquement acceptables topiques appropriés est de 0 % en poids à 10 % en poids.

8. La composition selon l'une quelconque des revendications 1 à 7, comprenant en outre un ou plusieurs excipients ou véhicules cosmétiquement acceptables topiques appropriés choisis parmi des stabilisants de la couleur, des colorants, des additifs, des filtres UV, des agents d'augmentation de la viscosité, des agents peptisants et des anti-oxydants.

9. La composition selon l'une quelconque des revendications 1 à 8, dans laquelle la quantité d'eau est d'au moins 4 % en poids à 18 % en poids.

10. La composition selon l'une quelconque des revendications 1 à 9, en forme d'une eau de toilette, eau de Cologne, eau de parfum, ou lotion après-rasage.
